# EUROPEAN PATENT SPECIFICATION

(11) **EP 1 444 518 B1**
(45) Date of publication and mention of the grant of the patent: **25.06.2008**
(21) Application number: 02784281.4
(22) Date of filing: 25.10.2002
(51) Int. Cl.: G01N 33/543

(54) **METHOD AND APPARATUS FOR MULTIPLEX FLOW CYTOMETRY ANALYSIS OF MIXED ANALYTES FROM BODILY FLUID SAMPLES**
VERFAHREN UND GERÄT FÜR MULTIPLEX DURCHFLUSSZYTOMETRISCHE BESTIMMUNG VON ANALYTEN IN PROBEN AUS KÖRPERLICHEN FLÜSSIGKEITEN
METHODE ET APPAREIL D'ANALYSE PAR CYTOMETRIE A FLUX MULTIPLEX DE SUBSTANCES A ANALYSER MELANGEES PROVENANT D'ECHANTILLONS DE FLUIDES CORPORELS

(30) Priority: 14.11.2001 US 991001
(43) Date of publication of application: 11.08.2004
(73) Proprietor: Beckman Coulter, Inc., Fullerton, CA 92834-3100 (US)
(72) Inventor: BELL, Michael, L., Fullerton, CA 92835 (US); MC NEAL, Jack, Fullerton, CA 90803 (US)
(74) Representative: Müller-Boré & Partner Patentanwälte
(86) International application number: PCT/US2002/034196
(87) International publication number: WO 2003/042698

(56) References cited:
- WO-A-99/64867
- US-A- 5 747 349
- US-A- 5 981 180
- US-A- 6 159 748

## Description

### BACKGROUND OF THE INVENTION

This invention relates to a reagent mixture, as well as to a method and apparatus, for carrying out simultaneous, automated analysis of multiple analytes in a test sample, particularly a bodily fluid. The invention is especially relevant to the field of general clinical chemistry, but may find application in other fields of use.

The medical condition of a patient is generally reflected by body chemistry. Accordingly, diagnostic medicine has developed a variety of analytical techniques to measure the presence and/or concentration of numerous test substances which serve as indicators of disease states or abnormalities. The great complexity of living organisms and the number and variety of substances for which detection and/or quantitative measurement may be helpful in identifying or excluding possible disease states or medical conditions has made it generally necessary to carry out a number of different tests. The need to carry out a number of different tests, however, has in turn increased the time and effort required to obtain meaningful test results, as well as the associated expense of analytical testing. In addition, the need to carry out multiple analytical tests may require the tester to obtain a large test sample or multiple samples, which may be difficult or impossible.

The sophistication of test procedures and the criticality of the test results also generally make it necessary to use highly trained personnel to carry out such assays, which in turn, also increases the cost of such testing. Even so, the possibility of human errors which could lead to inaccurate test results cannot be entirely excluded.

Attempts have been made to develop automated analysis systems. Conventional general chemistry systems assay one analyte at a time in bulk solution. The result of this type of single-test, single-assay design is that system complexity increases dramatically as the number of test samples (system throughput) and the number of analytes tested (system menu) increase. Since separate reagents are typically required for each test, it is necessary to maintain an inventory of a large number of different reagents, and a large number of separate reagent addition steps (e.g. pipetting steps) are required for each test series. Sequential execution of multiple assays also lengthens sample processing time.

More recently systems have been suggested to simultaneously analyze multiple analytes in a single test sample. See, for example, van den Engh et al., U.S. Patent No. 5,747,349. This patent proposes mixing different types of reporter beads with a test sample, with each type of reporter bead having an optical property which varies as a function of the concentration of a respective analyte in the sample, and measuring the optical properties of the reporter beads in a flow cytometer. Fluorescence and absorption are mentioned as optical properties which may be measured. The menu of analytes is limited, and details are not provided of how to assay for a number of important clinically relevant analytes, such as urea, creatinine, or many others. Thus, this system does not provide for carrying out a complete chemical analysis.

Another multiplex system is described in Chandler et al., U.S. Patent No. 5,981,180, entitled "Multiplexed Analysis of Clinical Specimens Apparatus and Methods." This document proposes flow cytometric analysis of a set of beads exposed to a test sample. It is contemplated that each member of the bead set would be designed to be individually distinguishable from the other members of the set and to react with a specific analyte of interest, which may be an antigen, antibody, peptide, protein, nucleic acid sequence and/or enzyme. In this system, the identity and quantity of each analyte of interest in the sample is determined by substantially contemporaneously collecting data relating to the classification of the particles, collecting data relating to the complexing or reaction of the particle with an analyte specific thereto, classifying the particles, and quantifying the amount of analyte associated with each particle in real time. Although this approach is theoretically promising in principle, it is difficult to put into actual practice, and the substantially contemporaneous real time analysis and quantification is subject to inaccuracies because it precludes statistical analysis of a distribution of values to exclude aberrant extreme values. Moreover the analyte palette is limited, and no provision is made for the analysis of electrolytes, such as alkali metal ions, or small metabolite molecules, such as glucose, which are important in numerous cases. Consequently, despite the extensive efforts of the prior art, it has continued to be necessary to carry out multiple tests, often with multiple test samples, and there has remained a need for a practical, automated system which is capable of reliably analyzing a broader spectrum of analytes of medical and/or biological interest in a single sample.

### SUMMARY OF THE INVENTION

It is an advantage of the present invention to provide an assay system, including an assay method, an assay apparatus and an assay reagent, which enables contemporaneous measurement of multiple analytes in a single test procedure.

Another advantage of the present invention is to provide a multiplexed assay system which is particularly applicable to carry out analysis of multiple routine general chemistry analytes including electrolytes and/or small metabolites in a bodily fluid sample.

A further advantage of the present invention is to provide an assay system which lends itself to miniaturization and which will facilitate the analysis of very small test samples.

It is also an advantage of the present invention to provide an assay system which is simpler and requires less maintenance than prior art systems.

Yet another advantage of the present invention is to provide an assay system which will minimize the number of reagents which must be inventoried by a laboratory in order to conduct a full analysis of a test sample.

A still further advantage of the present invention is to provide an assay system which can provide a complete analysis of a test sample in a cost effective manner.

Another advantage of the present invention is to provide an assay system which can carry out a complete chemical analysis of a clinical sample in less time and at lower overall cost that with prior art automated systems.

Still another advantage of the present invention is to provide an assay system which requires only one sample transfer step and one reagent transfer step to carry out a complete assay of multiple analytes in a clinical sample.

These and other advantages of the present invention are achieved by the method, apparatus and reagent mixture described and claimed hereinafter.

The present invention utilizes a reagent mixture or "cocktail" which includes different classes of particles or "beads" tailored to quantitatively detect specific analytes. This reagent particle mixture is incubated with a test sample for a sufficient time to permit the particles to complex or react with the respective analytes of interest, if those analytes are present in the sample. Then the reagent particle mixture is subjected to flow cytometry or to a similar technique that measures properties of individual particles to qualitatively and/or quantitatively measure the number of each type of particle and the proportion of each type of particle which has complexed or reacted with the specific analyte of interest to which it is directed.

The particles or beads themselves may be commercially available polystyrene latex particles which are functionalized to permit the attachment of binding agents or reactants for the analytes of interest and indicators or signaling agents for indicating the occurrence of binding or reaction with the analyte of interest. Also possible are acrylate or methacrylate derived particles produced by suspension or dispersion polymerization techniques. Other possible bead or particle sources include hydrogel polymer particles, polymerized micelle particles, particles produced by grinding cast films, particles produced by photopolymerization of aqueous emulsions, and particles produced by solvent casting as described in US patents 4,302,166 and 4,162,282. Bead sizes typically range from about 0.1 µm to about 50 µm, preferably from about 1 µm to about 20 µm. Their density is typically in the range of 0.5 to 2.0 grams per milliliter. This combination of size and density permits aqueous suspensions of the sensor beads to be handled as simple liquids which can be conveyed by fluid transfer apparatus such as pipettes, pumps, valves, etc. Thus, the particles are freely transportable in aqueous suspension by conventional fluid transfer techniques.

It may be advantageous for the beads or particles to include means (such as similar charges or surfactants) which effectively prevent interaction with other particulate sensors. This includes both prevention of the formation of particle agglomerates, which could clog the apparatus, and interference between particles of different types.

The particles should be sufficiently uniform in their properties that measurement of a reasonable number (i.e., less than 10,000) suffices to achieve clinically acceptable variance in assay results.

As used herein, a "type" of bead or particle refers to all particles which interact in the same way with a given analyte. It is, of course, possible for a reagent mixture to include two or more different types of particles which interact with the same analyte in different ways. Each type of particle incorporates coding indicia which enable unambiguous identification of the particle type, and consequently enables the analysis system to assign measurement signals from the particle to the specific analyte with which the bead interacts. Particle labeling or coding can be accomplished by varying detectable particle properties such as intensity of fluorescence from fluorescent dyes associated with the particles, ratios of intensities of fluorescence from multiple fluorescent dyes associated with the particles, fluorescence wavelengths of one or more fluorescent dyes associated with the particles, size of the particles, relative number of the particles, and combinations of any of the above characteristics. Of course, a "detectable signal" can be the absence of light, i.e., a fluorophore could be quenched so that it gives off no light. It is also possible to code the particles by adding materials with other detectable properties such as magnetic materials. Additional information about useful coding schemes may be found in Fulwyler, U.S. Patent No. 4,499,052; Coulter Electronics, UK Patent No. 1,561,042; and Tripatzis, European Patent No. 126450.

The first class of bead or particle is designed to measure electrolytes, i.e., ions present in the test sample. It is formed with alkyl acrylate, polyvinyl chloride or polystyrene beads in which are incorporated ionophores and other constituents to produce "optodes" that produce a signal upon binding of specific ions. Plasticized PVC beads have proven particularly suitable. Biologically relevant ions which may be specifically targeted by the "optode" particles include sodium, potassium, ammonium, calcium, chloride and carbon dioxide (carbonate). In addition to a label such as a particle size or one or more fluorescent markers or a combination of particle size and fluorescence marker(s) which identifies the type of particle, the beads also carry or include a fluorescent indicator or signaling agent which undergoes a shift in fluorescence emission wavelength or intensity when the ionophore molecules in or on the beads complex with an analyte ion of interest. The optodes are hydrophobic bulk phases containing detector ionophores, reporter ionophores and ionic sites. Ions from the aqueous sample diffuse into the organic phase and complex with the ionophores, which in turn transfer protons or other ions to preserve charge neutrality and produce a detectable fluorescence signal. Optode particles including useful ionophoric materials are disclosed in U. S. Patent No. 6,165,796 entitled "Pipetteable Ion Detector and Method. For potassium ions, for example, the ionophore may be Valinomycin, which is a known lipophilic, potassium-selective ionophore. A suitable ionophore for ammonia is monactin; for sodium is N,N,N=,N=-tetracyclohexyl-1,2-phenylenedioxydiacetamide (ETH2120); for calcium is ETH1001, and for chloride is chloro(octaethylporphyrinato)indium. By using a flow cytometer to detect the fluorescence emission wavelength or intensity, it can be determined whether the particles have complexed with target ions, and the quantity of such ions present in the test sample can be determined. The ionophore and the indicator may be parts of a single molecule (one-component system) or the ionophore and the indicator may be separate molecules distributed uniformly on or through the particles (two-component system).

The second class of particle is designed to measure small metabolite molecules, e.g. saccharides such as glucose, fructose, lactose or galactose. Other small metabolite molecules of interest to measure include, for example, ammonia, urea, uric acid, cholesterol, triglycerides, ethanol, lactate, salicylate, acetaminophen, bilirubin and creatinine. Like the other particles, the particles of this second class each has an identifying label which uniquely identifies the particular type of particle it is. In addition, it has immobilized thereon a signaling ligand molecule which binds specifically to the analyte of interest and which becomes fluorescent when it binds to the analyte, for example, to glucose. Examples of signaling ligands of this type are disclosed in U.S. Patent Nos. 5,503,770 and 5,763,238. A further example is disclosed in co-pending U.S. Patent Application entitled Photo-Induced Electron Transfer Fluorescent Sensor Molecules which is commonly assigned herewith. Aqueous phase ligands can be bound to the surface of hydrophobic particles or distributed internally in hydrophilic particles. Ligands that require an organic phase can be distributed inside hydrophobic particles. A particularly preferred signaling ligand molecule for saccharides, such as glucose, comprises a dual phenyl boronate structure coupled by a non-fluorescent spacing linker region through two tertiary amine groups. One of the tertiary amine groups also is used to attach a structural group which changes its fluorescent properties, e.g., becomes fluorescent and responds to excitation by light in the near-infrared region, when a saccharide molecule is complexed with the boronate groups. When the boronic acid groups bond to the cis diols on the saccharide, the resulting increased acidity of the boronic acid pulls charge away from the fluor, thereby increasing fluorescence. In this way, fluorescent signal generation is tied to analyte binding. The other tertiary amino group is used to connect to an anchor group which immobilizes the signaling ligand molecule on the particle. The specificity of the signaling ligand for different analytes can be varied by adjusting the size and/or configuration of the spacing linker to adapt the spacing and orientation of the boronate groups to match the analyte of interest.

An alternative sensor particle for the analysis of small metabolite molecules uses non-signaling ligands and labeled analogues to metabolites in a competitive binding assay format. The non-signaling ligands closely resemble the signaling ligands, but lack the fluorescent group. These ligands bind either to the analyte or to the labeled analogue in proportion to their relative concentrations in the reaction mixture. The amount of label bound to the particles is thus inversely related to the amount of sample in the reaction mixture. The particulate sensors have diameters in the range of 0.1 micrometer to 50 micrometers, and preferably in the range of 1 micrometer to 20 micrometers. Their density is within the range of 0.5 to 2.0 grams per milliliter. This range is easily achievable by forming the sensors from plastics such as polystyrene or polyvinyl chloride. This combination of size and density permits the suspended sensors to be treated as simple liquids by most fluid transfer apparatus such as pipettes, pumps, and certain valves.

The particulate sensors produce a signal in response to the analyte of interest by reversibly binding the analyte. A labeled analog of the analyte competes with the analyte for a limited number of binding sites. The amount of the detectable labeled analog bound is inversely related to the concentration of analyte in the reaction mixture. This procedure is exemplified in the example concerning glucose sensing particles described above.

The third class of particle is designed to measure enzymes, i.e. proteins which bind to receptor molecules and mediate biological activities. Illustrative examples of enzymes which can be measured include alkaline phosphatase, alanine aminotransferase, aspartate aminotransferase, amylase, cholinesterase, creatine kinase, gamma-glutamyl transferase, lactate dehydrogenase and lipase. Suitable materials for this third class of particle include, without limitation, "latex" particles or polystyrene beads. Again, in addition to a particle label which uniquely identifies the particular type of particle as described above, the particles have immobilized thereon a fluorogenic substrate which generates a signal in response to the activity of the target enzyme. The fluorogenic substrate may be a molecule which includes a specific binding site for an enzyme analyte of interest and which undergoes a shift in fluorescence properties when it binds to a protein, or it may be a substance which undergoes a shift in fluorescence properties when acted upon by an enzyme. For example, the serum enzyme alkaline phosphatase may be measured with beads incorporating 4-methylumbilliferone phosphate as an indicating or signaling agent. Charge transfer which occurs when this initially non-fluorescent material is dephosphorylated by alkaline phosphatase renders the resulting molecule fluorescent. Another example is the enzyme alpha-amylase, which may be detected using an indicator substrate comprised of maltopentaose with 3-(2-hydroxyethyl)indole and 5-(2-aminoethylamino)-1-naphthalene sulfonate fluorescent structures attached to the ends as described in Payre et al., Angew. Chem. Int. Ed. Engl. 34(11):1239-41 (1995). This substrate may be coupled at the nonreducing end of the saccharide to latex particles. Cleavage of the saccharide chain by the amylase enzyme prevents resonance energy transfer between the dye groups and produces a marked decrease in fluorescence. As with the other classes of particles, the identities of the particular particles and the proportion, if any, which is bound to the enzyme analyte of interest can be determined by flow cytometry or by similar techniques that measure properties of particles individually.

The fourth class of particle is designed to measure antibodies or antigens. Like the other classes of particles, these particles are uniquely labeled by size or one or more fluorescent dyes or both so that the type of particle can be identified by flow cytometry. The particles further have a specific antibody immobilized thereon if it is desired to measure the presence of an antigen of interest in the test sample, or if it is desired to measure the presence of an antibody in the sample, then the particles have immobilized thereon an antigen with which the antibody of interest specifically binds. Examples of antigens which may be detected include myoglobin, troponin I and CK-MB. An analogue of the antigen or antibody to be measured labeled with a fluorescent label can be added to the system, and the antigen or antibody assay can be carried out as a competitive binding assay, or alternatively, a second antibody which binds to a different epitope on the antigen or antibody and which is labeled with a fluorescent label can be added to the system, and the assay can be carried out as a sandwich assay. The competitive immunoassay approach is preferred for smaller molecules because the sandwich assay approach requires the presence of two binding sites on the molecule, which may not be present or which may be sterically hindered in a smaller molecule. Examples of useful antibody/antigen beads are disclosed in Saunders, U.S. Patent No. 4,665,020 and in Fert et al., co-pending U.S. Patent Application No. 09/154,248.

A fifth class of particle which may be present in the reagent mixture is designed to detect and/or measure a specific polynucleotide sequence of interest, e.g., a gene or a messenger RNA. Such particles may be useful to test for the presence of a virus, such as the HIV, or for a genetic mutation, such as one of the mutations which causes cystic fibrosis. These particles, besides being labeled so that the type of each particle can be distinguished from other types of particles present in the reagent mixture, also have a polynucleotide sequence immobilized thereon which is complementary to, and which consequently under appropriate hybridization conditions can hybridize with, the polynucleotide sequence of interest. The system also contains a fluorescently labeled tag which binds to a double strand of DNA, but not to a single DNA strand, and which therefore enables a flow cytometer to distinguish between beads which carry hybridized DNA sequences and unreacted beads carrying only the immobilized single polynucleotide strand. Alternatively, an analogue of the polynucleotide sequence of interest labeled with a fluorescent label may be added to the system and the assay carried out as a competitive binding assay. Examples of particles specifically adapted for analysis of DNA sequences are disclosed in Fulton, U.S. Patent Nos. 5,736,330 and 6,057,107.

The reagent mixture of the invention may contain more than one type of particle from any of the foregoing general classes. For example, the reagent mixture may contain beads designed to quantitatively measure the presence of potassium ions in a sample together with other particles designed to quantitatively measure calcium ions, or the reagent mixture may contain beads designed to measure the amount of glucose present in a sample together with other particles which measure urea.

This invention contemplates that the reagent mixture of the invention contain beads from each of the first two classes enumerated above. In general, because of the importance of assays for electrolytes and metabolites, the reagent mixtures of the invention will contain at least one type of bead selected from each of these classes. At least one bead from each of the classes directed to measurement of enzymes, antibodies or antigens, and/or DNA sequences is to be included. It should be understood that it is the intent of the invention to provide a reagent mixture containing beads designed to measure all analytes of common interest, so that a single test using only one reagent mixture can be used for all analyses, and it will not be necessary to carry out multiple tests, to maintain separate supplies of different reagent mixtures or to switch reagent solutions used in the flow cytometer. In a preferred embodiment, the reagent mixture of the invention contains beads from all of the five classes enumerated above.

Known fluorescent dyes can be used to label individual types of beads for identification by the flow cytometer. Examples of suitable fluorescent dyes which are hydrophobic and stable and can be used for this purpose include those known by the designations IR792 ([2-[2-[3-[(1,3-dihydro-3,3-dimethyl-1-propyl-2H-indol-2-ylidine)ethylidine]-2-(phenylthio)-1-cyclohexen-1-yl]ethenyl]-3,3-dimethyl-1-propylindolium perchlorate]), IR768 ([2-[2-[3-[(1,3-dihydro-3,3-dimethyl-1-propyl-2H-indol-2-ylidine)ethylidine]-2-phenoxy-1-cyclohexen-1-yl]ethenyl]-3,3-dimethyl-1-propylindolium perchlorate])YL22 ([2-[2-[3-[(1,3-dihydro-3,3-dimethyl-1-propyl-2H-benzoindol-2-ylidine)ethylidine]-2-(phenylthio)-1-cyclohexen-1-yl]etbenyl]-3,3-dimethyl-1-propylbenzoindolium iodide]), IR780 ([2-[2-[2-chloro-3-[(1,3-dihydro-3,3-dimethyl-1-propyl-2H-indol-2-ylidine)ethylidine]-1-cyaohexen-1-yl]ethenyl]-3,3-dimethyl-1-propylindolium perchlorate]), JM5488-48 ([2-[2-[2-chloro-3-[(1,3-dihydro-3,3-dimethyl-1-decanyl-2H-benzoindol-2-ylidine)ethylidine]-1-cylohexen-1-yl]ethenyl]-3,3-dimethyl-1-decanylbenzoindolium iodide]), JM5488-72 ([2-[2-[-3-[(1,3-dihydro-3,3-dimethyl-1-decanyl-2H-benzoindol-2-ylidine)ethylidine]-1-cyclohexen-1-yl]ethenyl]-3,3-dimethyl-1-decanylbenzoindolium iodide]) IR140 and IR143 and their derivatives. Individual dyes or combinations of dyes in varying amounts or ratios can be used, optionally in conjunction with particle size characteristics, to provide a unique identifier for each type of particle in the bead mixture. This is described more fully in a co-pending application WO03/042695 entitled "Analyte Detection Systems, by Bell et al.

Preferred dye structures which may be used to indicate complexing or reaction with an analyte of interest include the cyanine dyes known under the designations Cy5, dibenzoCy5, Cy7 and dibenzoCy7 as well as Nile Blue derivatives such as ETH 5294. It is not necessary for these dyes to be unique to each type of bead in the reagent mixture. Rather, since the different types of beads are themselves each uniquely labeled for identification, the same dye can be used on several different types of beads to indicate whether that bead is complexed, or has reacted, with its respective analyte of interest.

Further examples of useful fluorescent dyes for labeling individual types of beads or for indicating complexing or reaction with an analyte of interest are known in the art. See, for example, R. P. Haugland, Handbook of Fluorescent Probes and Research Chemicals, 5th Edition, Molecular Probes Inc., Eugene, 1992).

In use, the reagent admixture is suspended in an aqueous suspension. Such suspensions can be readily handled by pipetting. It is desirable to buffer the reagent bead mixture to assure stable test conditions. In this regard, it is preferred to use a buffer such as HEPES buffer, which contains no sodium, phosphate or urea which would interfere with the analysis of electrolytes and/or small metabolite molecules.

The preferred technique for reading the assays according to the invention is flow cytometry in which the particles are read individually as they flow through a reading device. When read, the particles are both identified by their coding indicia and measured for interaction with their respective analytes. The measured results thus can be appropriately allocated to the analyte to which they pertain. This permits mixing of reagent particles directed to different analytes in a single reagent mixture and simultaneous analysis of multiple analytes in a single test sample.

Because flow cytometry is readily able to distinguish between the reagent beads suspended in the test solution and other components of the test solution, the flow cytometer used to carry out the assay procedure of the invention can differentiate between fluorescent structures bound to test beads and those same or similar structures present in unbound form in the test medium. Consequently, it is not essential to wash the beads after incubation with the test sample and before passage through the flow cytometer. This represents a valuable simplification of the assay of the present invention compared to prior art procedures which require substrate washing to remove excess reagent before analysis can take place.

The apparatus used to carry out the invention comprises a known means for introducing a test sample which is to be analyzed; means for introducing a reagent bead mixture containing beads designed to measure each analyte of interest, an incubation chamber or vessel in which the sample and reagent mixture are allowed to react, and a flow cytometer read cell for reading the bead label of each bead passed through the cell to identify the type of bead and analyte of interest and for reading the fluorescent indicator associated with each bead to determine the extent that the bead has complexed or reacted with the respective analyte of interest. Optionally, the apparatus may also include a washing apparatus for washing the beads between incubation with the test sample and passage through the read cell, but as noted above, this is not essential. The read cell includes a first laser or coding laser and a first detector for reading the bead label or coding in order to determine the type of bead and analyte of interest, and it may also include a second laser or assay laser and second detector for reading the fluorescent indicator associated with each bead. Alternatively, a single laser may perform both functions. Additional detectors may be associated with each laser. Such additional detectors would be sensitive to light within a limited range of wavelengths so that signals from different fluorescent dyes may be distinguished. A 15 mW diode laser emitting light at a wavelength of about 635 nm, for example, can be used to good effect as the second laser. The first laser advantageously may be, for example; a 30 mW diode laser emitting light at a wavelength of about 785 nm. These wavelengths lie in the near-infrared wavelength range and are somewhat longer than usual in flow cytometry, but are particularly useful in the present invention because they avoid potential interference in the visible light range caused by substances naturally present in serum or other biological samples. Operation in the near-infrared wavelength range also facilitates the use of laser diodes, which are significantly less expensive than conventional gas lasers. The detectors may be photomultipliers or photodiodes. Photodiodes are preferred both because their solid state nature facilitates miniaturization and operational reliability and because they work better at longer wavelengths in the near-infrared range.

As is known in flow cytometry, the forward scatter and side scatter signals can be used singly or together to determine particle size.

Examples of test samples which may be analyzed by the method and apparatus of the invention using the inventive reagent mixtures include whole blood, serum, plasma, lymph, spinal fluid, tears, mucous, saliva, semen, urine and other bodily fluids.

The method of the invention generally involves the steps of:
- admixing a test sample containing multiple analytes to be measured with a reagent mixture comprising a plurality of types of sensor particles, each type of sensor particle comprising coding indicia which confer uniquely identifying optical properties on that type of particle and a measurement substrate which specifically interacts with an analyte of interest, said reagent mixture including particles which interact specifically with each of the following analytes:
   (A) at least one analyte selected from the group consisting of alkali metal ions, alkaline earth metal ions, ammonium, halide ions, oxygen, pH, carbon dioxide;
   (B) at least one analyte selected from the group consisting of saccharides, ammonia, urea, uric acid, cholesterol, triglycerides, ethanol, lactate, salicylate, acetaminophen, bilirubin and creatinine;
   (C) at least one analyte selected from the group consisting of enzymes, antibodies, antigens and polynucleotide sequences;
- allowing the resulting admixture to incubate for a period of time sufficient for each type of sensor particle to interact with the analyte to which it is directed to affect the measurement optical properties of the sensor particle;
- transferring the admixture to a reading device and reading both the coding and the measurement optical properties of each sensor particle individually;
- storing the measured optical properties of each sensor particle type according to the coding optical properties read from the particles, and
- processing the stored measurements for each sensor particle type to obtain an assay result for the analyte associated with each type of sensor particle; whereby a complete chemical analysis of the test sample is obtained.

The alkali metal ions are preferably selected from the group consisting of ions of sodium and potassium; the alkaline earth metal ions are selected from the group consisting of ions of calcium and magnesium; and the halide ions are chloride ions. The saccharides are selected from the group consisting of glucose, fructose, lactose and galactose.

The enzymes are selected from the group consisting of alkaline phosphatase, alanine aminotransferase, aspartate aminotransferase, amylase, cholinesterase, creatine kinase, gamma-glutamyl transferase, lactate dehydrogenase and lipase.

The most preferred analytes are sodium, potassium, chloride, glucose, urea, creatinine, albumin, creatine kinase 1, troponin I, and myoglobin

The process and apparatus of the invention have the advantages that the apparatus can be miniaturized to save space and weight. The system is also comparatively simple compared to other types of assay systems, which increases reliability and decreases maintenance costs. Moreover, due to the great sensitivity of the method and apparatus, it is possible to carry out complete sample assays with lesser amounts of reagent than required by prior art systems.

Other objects, advantages and novel features of the present invention will become apparent from the following detailed description of the invention when considered in conjunction with the accompanying drawings.

### BRIEF DESCRIPTION OF THE DRAWING

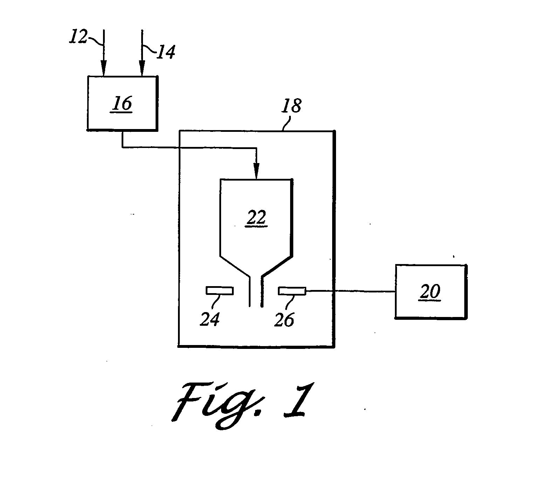

Fig. 1 is a schematic depiction of the apparatus of the instant invention.

### DETAILED DESCRIPTION OF THE PREFERRED EMBODIMENTS

The preferred embodiments of the instant invention are best understood by reference to the following example.

### EXAMPLE

In this example, a test sample is prepared using a mixture of analytes as listed in the "menu."

Menu: sodium, potassium, glucose, α-amylase, Thyroid Stimulating Hormone (TSH)

The procedure includes the following steps:
- Prepare each sensor particle type and each support reagent separately;
- Mix aliquots of the components to form a combined reagent;
- Mix aliquots of the combined reagent with calibrators and with test samples;
- Measure the results of the reactions; and
- Combine the measured results to determine the concentration of analytes in the test sample.

### PREPARATION OF SENSING PARTICLES:

### Sodium Ion Sensing Particles:

Prepare sodium ion sensing particles by solvent casting as described in U.S. Patents 4,302,166 and 4,162,282.

Combine:
15 mg Sodium Ionophore X (4- tert-butylcalix[4]arene-tetraacetic acid tetraethyl ester),
1.75 mg ETH 5294 (9-(diethylamino)-5-(octadecanoylimino)-5H-benzo[a]phenoxazine),
4.1 mg sodium tetrakis [3,5-Bis (trifluoromethyl)phenyl]borate,
50 mg high molecular weight polyvinyl chloride, and
100 mg Bis(2-ethylhexyl) sebacate.

The above materials are available from Fluka Chemical Corp. of Milwaukee, Wisconsin.

Dissolve in:
5 ml cyclohexanone
Dilute the solution with:
1 ml xylene
200 ml dichloromethane

Cast particles at a pulse rate of 17,500 Hz using a core flow rate of 0.5 milliliter per minute through a 0.0015" diameter orifice with a deionized water sheath flowing at 54 milliliters per minute into a 2 liter vessel to which a 3% solution of polyethylene glycol (MW 600, Polysciences, Inc. of Warrington, Pennsylvania) flows dropwise at a rate of 20 microliters per minute. Catch the overflow in a 20 liter vessel and allow the particles to settle for 24 hours. Decant the bulk of the liquid and resuspend the particles in 4 mM Tris with 0.01 % Tween 20 at pH 7.4.

### Potassium Ion Sensing Particles:

Prepare potassium ion sensing particles by solvent casting as described in US patents 4,302,166 and 4,162,282.

Combine:
9 mg BME-44 (2-dodecyl-2-methyl-1,3-propanediyl bis[N-[5=-nitro(benzo-15-crown-5)-4=-yl] carbamate]),
6 mg ETH 5294 (9-(diethylamino)-5-(octadecanoylimino)-5H-benzo[a] phenoxazine),
7.5 mg sodium tetrakis [3,5-Bis (trifluoromethyl) phenyl] borate,
60 mg high molecular weight polyvinyl chloride, and
120 mg Bis (2-ethylhexyl) sebacate,
The above materials are available from Fluka Chemical Corp. of Milwaukee,
Wisconsin.
Dissolve in:
5 ml cyclohexanone
Dilute the solution with:
1 ml xylene
200 ml dichloromethane

Cast particles at a pulse rate of 25,000 Hz using a core flow rate of 0.2 milliliter per minute through a 0.0015" diameter orifice with a deionized water sheath flowing at 70 milliliters per minute into a 2 liter vessel to which a 3% solution of polyethylene glycol (MW 600, Polysciences, Inc. of Warrington, Pennsylvania) flows dropwise at a rate of 20 microliters per minute. Catch the overflow in a 20 liter vessel and allow the particles to settle for 24 hours. Decant the bulk of the liquid and resuspend the particles in 4 mM Tris with 0.01 % Tween 20 at pH 7.4.

### Glucose sensing particles:

Produce glucose sensitive particles by synthesizing a signaling ligand onto the surface of carboxylated latex particles. This ligand has high selectivity for glucose and changes its fluorescence in response to glucose binding. The synthesis is described below; the polymer shown (5) is in the form of 3.2 micrometer diameter poly(styrene/5.5% divinyl benzene/5% methacrylic acid) beads, which are available from Bangs Laboratories, Inc. of Fishers, Indiana.

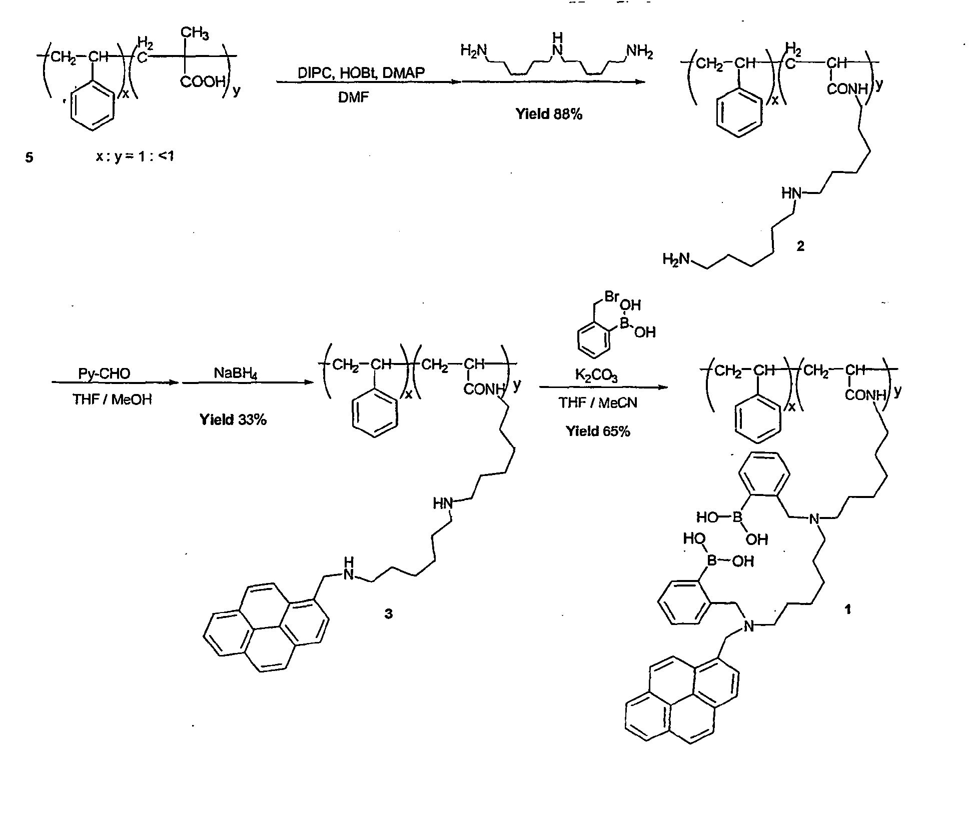

### Preparation of 2

Stir a suspension of cross-linked carboxylate modified polymer beads (200 mg), 1,3-diisopropylcarbodiimide (DIPC) (170 mg), 1-hydroxy-1H-benzotriazol (HOBt) (200 mg,), 4-*N,N*-dimethylaminopyridine (DMAP) (8 mg) in *N,*N-dimethylformamide (DMF) (5 ml) on an ice bath under nitrogen atmosphere for 30 minutes. Add bis (hexamethylene) triamine (1.0 g) to the solution and stir at room temperature for 1 day. Pour this reaction mixture into tetrahydrofuran (THF) and stir for 1 hour at room temperature. Centrifuge to settle the beads, decant the supernatant, and resuspend in methanol. Repeat the centrifugation and decanting, then resuspend in methanol.

### Preparation of 3

Stir a suspension of 2 (200 mg) and and 1-pyrenecarboxyaldehyde (116 mg, 0.50 mmol) in THF and methanol (7 ml each) at room temperature for 5 hours. Add sodium borohydride (57 mg) to the reaction, and then stir at room temperature for 1 hour. Remove the solvent under reduced pressure and suspend the residue in chloroform. Wash with water, dry over magnesium sulfate, and remove the solvent under reduced pressure. Wash the residue twice in methanol by centrifugation, decanting, and resuspension.

### Preparation of 1

Heat a suspension of 3 (95 mg), potassium carbonate (116 mg) and 2-(2-bromobenzyl)-1,3-dioxa-2-borinane (93 mg) in THF (5 ml) and acetonitrile (3 ml) at reflux for 7 hours. Cool to room temperature, then remove under reduced pressure. Suspend the residue in chloroform, and wash with water. Dry over magnesium sulfate, then remove the solvent under reduced pressure. Resuspend the residue in chloroform and n-hexane, then wash by centrifuging and decanting. Resuspend the washed beads in 50 mM Tris buffer adjusted to pH 7.2.

### Amylase Sensing Particles:

Human serum α-amylase cleaves modified maltopentaose substrates with good specificity. Maltopentaose can be labeled with a fluorescence acceptor molecule at one end of the oligosaccharide and a fluorescence donor molecule at the opposite end. An α-amylase enzyme substrate prepared in this way showed a fluorescence intensity change as the enzyme digested the substrate. Nathalie Payre, Sylvain Cottaz, and Hugues Driguez used 5-(2-amionoethylamino)-1-naphthalene-sulfonate (EDANS) as an acceptor and 3-(2-hydroxyethyl)indole as donor. This produced a fluorescence decrease as the donor was cleaved away from the acceptor by α-amylase (Angv. Chem. Intl. Ed. Engl. 1995, 34 (No. 11)). Edmund D. Matayoshi, Gary T. Wang, Grant A. Krafet, and John Erickson further showed an octapeptide labeled at opposite ends with DABCYL (4-(4-Dimethylaminophenylazo)benzoic acid), a fluorescence absorbing acceptor and EDANS, a fluorescence emitting donor, i.e. DABCYL-SerGlnAsnTyrProlleValGln-EDANS. This quenched fluorogenic substrate showed increasing fluorescence as a protease enzyme cleaved the octapeptide. (Science, Vol. 247, 954(1990). Such an increasing signal is desirable to improve sensitivity and precision.

Robert H. Fairclough and Charles R. Cantor, list many intramolecular energy transfer pairs that may be suitable for similar methods (Methods in Enzymology. 48, 347(1978)).

To produce α-amylase sensitive particles that have this preferred direction of signal change, EDANS was selected as fluorescence donor and DABCYL as fluorescence quenching acceptor moieties. These are covalently attached to an α-amylase substrate, pentaose, and spatially disposed less than 100 Angstroms apart to facilitate fluorescence energy transfer. This substrate was designated as a "quenched fluorogenic enzyme substrate". The quenched fluorogenic enzyme substrate is, in turn, attached to latex particles via amide bond formation between a carboxylic acid moiety of the latex particles and the amino terminal of the aliphatic pendant group of the fluorogenic enzyme substrate molecule. Care must be taken in selecting the attachment point of the substrate molecule. One should attach the fluorescent moiety to the solid phase. Thus, upon enzyme cleavage of the oligosaccharide, the "fluorescence quencher" member of the donor/acceptor pair is released into the bulk solution, and the remaining "bare" fluorescent moiety fluoresces on the solid phase. A flow cytometer can enumerate those particles, quantitate the fluorescence intensity, and relate it to the α-amylase activity.
DABCYL-OLIGOSACCHARIDE-EDANS as an exemplar compound for a quenched fluorogenic enzyme substrate for α-amylase. This substrate is attached to polymeric particles with carboxylic acid surface functional groups. The key intermediate is obtained by the method of US Patent 5,851,778, *Analyte Assay Using A Trifunctional Conjugate.* Details of the synthesis are: 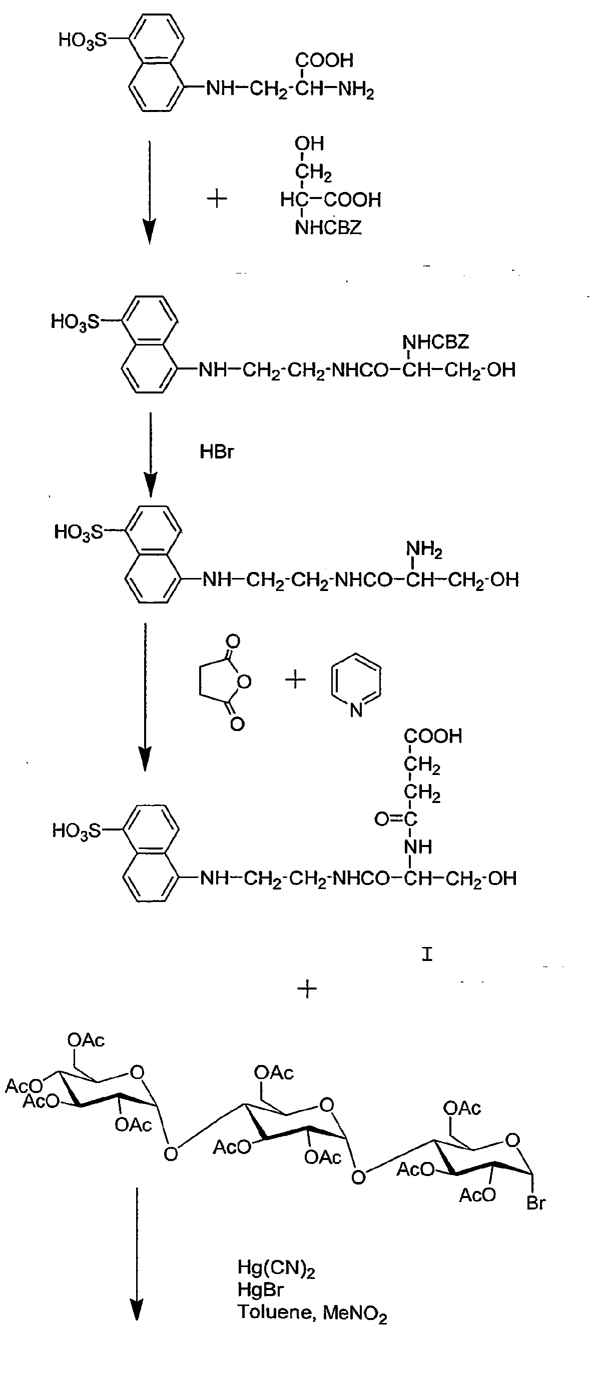 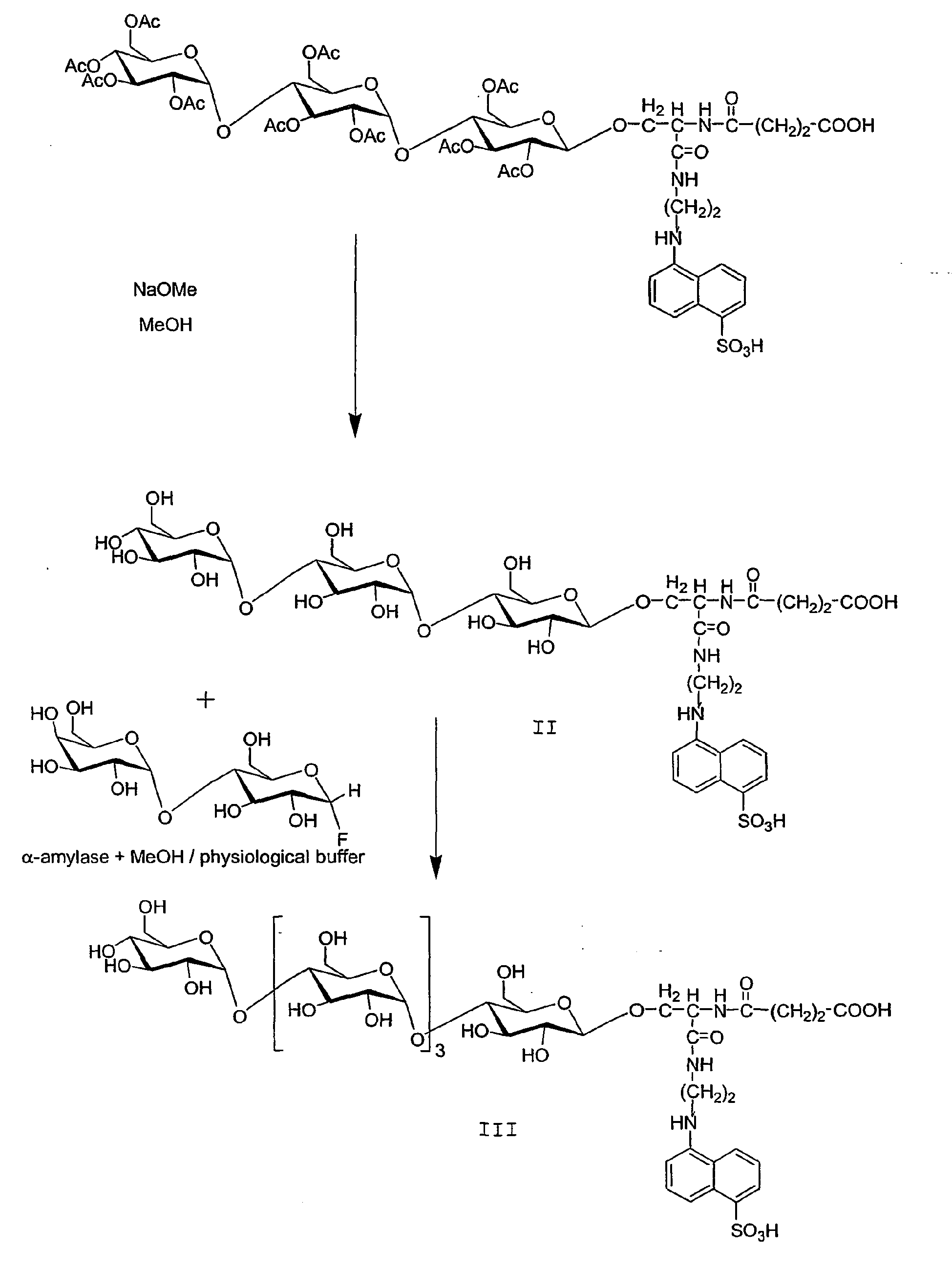 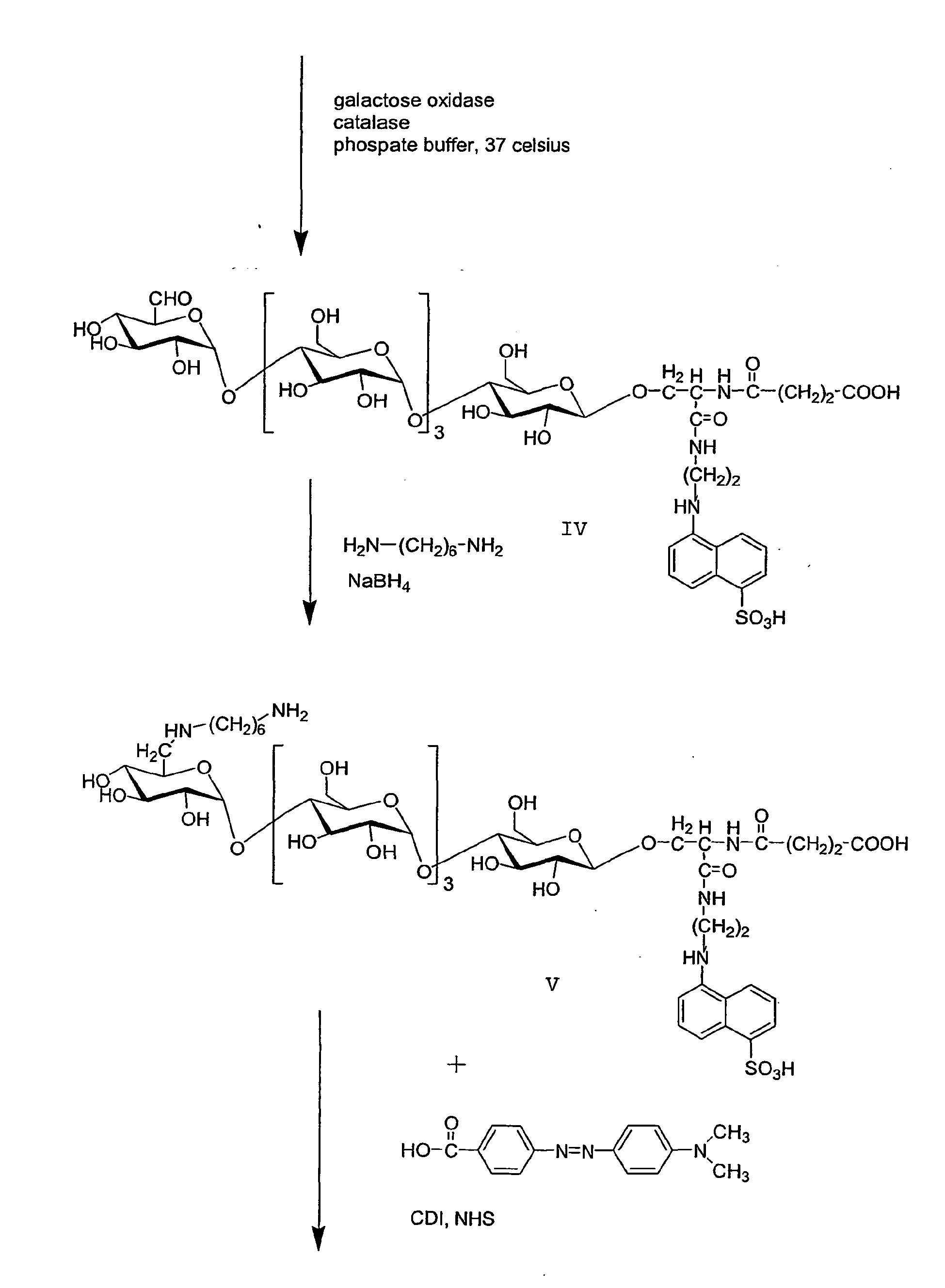 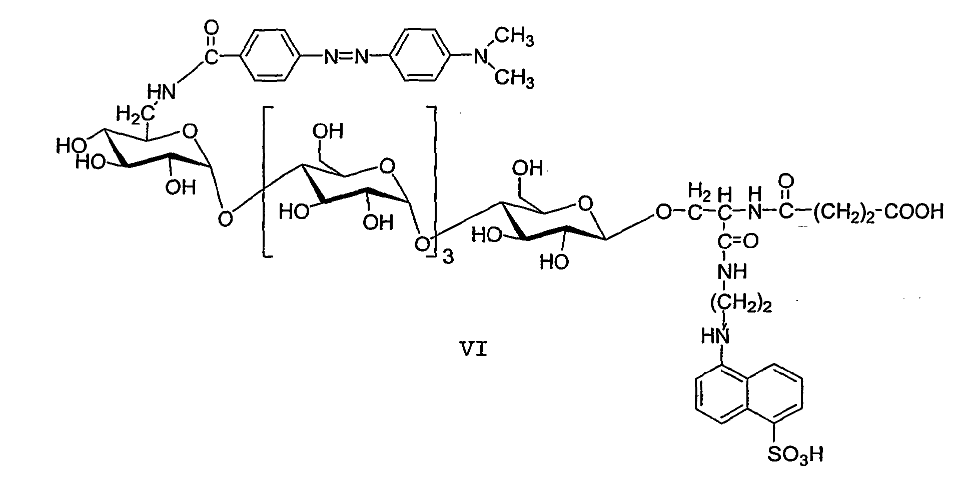
***1. Preparation of Hydroxymethyl-EDANS (I):***
   Activate carbobenzoxy-protected-Serine with Carbonyl-diimidazole (CDI) in DMF or Dicyclohexylcarbodiimidazole (DDCI) and react with 5-[(2-Aminoethyl)amino]napthalene-1-sulfonic acid (EDANS). Remove the carbobenzoxy protecting group by treatment with hydrobromic acid or catalytic hydrogenation to obtain the amino-terminated compound. Convert the amino-functional group to carboxy terminus by treating with succinic anhydride to obtain I.
***2. Trisaccharide-EDANS (II):***
   React I with Bromo-trisaccharide with Hg(CN)₂, HgBr, in Toluene/Nitromethane as described by Nathalie Payre, Sylvain Cottaz, and Hugues Driguez in Angv. Chem. Intl. Ed. Engl. 1995, 34 (No. 11). De-acetylate the product with Sodium Methoxide in Methanol to obtain II.
***3. Pentasaccharide-EDANS (III):***
   Couple II to fluoro-disaccharide with α-Amylase in Methanol/Phosphate Buffer as described by Nathalie Payre, Sylvain Cottaz, and Hugues Driguez in Angv. Chem. Intl. Ed. Engl. 1995, 34 (No. 11) to obtain pentasaccharide-EDANS, III.
***4**.* Oxidize the 5-hydroxymethyl group of the terminal saccharide enzymatically by Galactose oxidase and Catalase in Phosphate Buffer at 37°C to yield the aldehyde, IV, according to the method of Nathalie Payre, Sylvain Cottaz, and Hugues Driguez in Angv. Chem. Intl. Ed. Engl. 1995, 34 (No. 11).
***5***. React the aldehyde group of IV with 1,6-Hexanediamine and reduce the subsequent Schiff's Base with Sodium Borohydride to yield the Amino-pentasacchride-EDANS, V.
***6***. Activate the carboxylic acid group of 4-[(4-Dimethylamino)-phenylazo]benzoic acid with CDI or DCCI and react with the above primary amino group of V to yield the product VI.
***7***. Bind VI to the surface of latex particles by activating the particles with EDAC, binding a 1,6-hexanediamine spacer to the particles, activating VI with CDI, and binding the activated form of VI to the distal end of the spacer.
   7.1 Latex activation. Wash 10 mg of 4.4 micrometer diameter carboxylate modified polystyrene latex particles (Bangs Laboratories, Indianapolis, IN) in 1 ml of 50 mM MES buffer, pH 6.1. Repeat wash and resuspend by sonication in 1 ml of MES buffer. Add 10 mg of EDAC and react with continuous mixing for 15 minutes at 20° C. Wash the activated particles twice again in 50 mM MES buffer adjusted to pH 7.8. Resuspend by sonication in 0.5 ml of 50mM MES buffer pH 7.8.
   7.2 Spacer binding. Add 0.5 ml of 1 mM 1,6-hexanediamine in 50 mM MES buffer pH 7.8 and react with continuous mixing for 3 hours. Wash and resuspend in 1 ml of 0.03% glycine in 50 mM MES buffer for 30 minutes with gentle mixing. Wash the particles twice again in 50 mM MES buffer adjusted to pH 7.8 to remove residual amine. Resuspend by sonication in 0.5 ml of 50 mM MES buffer pH 7.8.
   *7.3* VI activation. Activate the carboxylic acid group of VI with CDI in DMF and add to the latex suspension with continuous mixing for 3 hours. Wash and resuspend in 1 ml of 0.03% glycine in 50mM MES buffer for 30 minutes with gentle mixing. Wash the particles twice again in 50 mM Tris buffer adjusted to pH 7.2. Resuspend the washed beads by with sonication in 50 mM Tris buffer adjusted to pH 7.2.

### TSH Capture Particles:

Wash 10 mg of 5.6 micrometer diameter carboxylate modified polystyrene latex particles (Bangs Laboratories, Indianapolis, IN) in 1 ml of 50mM MES buffer, pH 6.1. Repeat wash and resuspend by sonication in 1 ml of MES buffer. Add 10 mg of EDAC (1-Ethyl-3- (3-dimethylaminopropyl)-carbodiimide hydrochloride) and react with continuous mixing for 15 minutes at 20° C. Wash the activated particles twice again in 50 mM MES buffer adjusted to pH 7.8. Resuspend by sonication in 0.5 ml of 50mM MES buffer pH 7.8. Add 1.8 mg mouse monoclonal anti-human TSH antibody (BioCheck, Inc. Burlingame, CA) in 0.5 ml of 50mM MES buffer pH 7.8 and react with continuous mixing for 3 hours. Wash and resuspend in 1 ml of 0.03% glycine in 50mM MES buffer for 30 minutes with gentle mixing. Wash and resuspend in 1% bovine serum albumin in 4mM Tris buffer at pH 7.4.

### SUPPORT REAGENTS:

### TSH Reporter Antibody

Conjugate 1 mg of affinity purified goat anti-human TSH antibody (BioCheck, Inc. Burlingame, CA) to Cy5 dye (Fluorolink Cy5, Amersham Pharmacia Biotech Inc. of Piscataway, NJ) according to the dye manufacturer's directions. Suspend the labeled antibody (after purification by gel permeation chromatography) in 10 ml Tris buffer at pH 7.4.

### Combined Reagent:

Measure a dilute aliquot of each particle type in the flow cytometer described below to establish signature values for forward and side scatter. Produce a combined reagent by mixing equal numbers of each type of analyte bead to a combined concentration of 250,000 beads per ml in 5 mM Tris buffer with 0.01% Tween 20 adjusted to pH 7.4. Add TSH reporter Ab to a final concentration of 12 micrograms per ml.

### React Combined Reagent:

Prepare six calibrator solutions by adding menu analytes spanning from one half the minimum to twice the maximum of the reference clinical concentration interval for each analyte. Mix 20 microliter aliquots of the combined reagent in separate reaction vessels with 20 microliters of each calibrator solution and any test samples. Add 160 microliters of Tris buffer at pH 7.4 to each reaction vessel. Maintain the temperature within 0.2 degrees of 37 Celsius. Stir gently for 30 minutes before measuring results.

### Measure Results of Reactions:

Measure particle signals using a flow cytometer with confocal excitation at 633 nm and 340 nm. Detect forward scatter and side scatter from the 633 nm source. Detect fluorescence in two channels with wavelength sensitivity of 450 to 520 nm in the first fluorescence detection channel and of 650 to 720 nm in the second fluorescence detection channel. Trigger data collection on the forward scatter signal. Group the particle detected into clusters of similar forward and side scatter. This distinguishes the particle types, which are of different sizes.

### Combined the measured results:

Associate the measured clusters of similar forward and side scatter to analyte specificity by reference to the scatter measurements made prior to forming the combined analyte. For each reaction, for each analyte-associated cluster, calculate a value equal to the median fluorescence from the fluorescence detection channel associated with the signal of that analyte. For α-amylase activity, the appropriate signal is that from the first fluorescence detection channel. For all other analytes, the appropriate signal is that from the second fluorescence channel. This produces a set of five values for each reaction. Each value is associated with one of the five analytes in the test menu. Determine the relationship between the values and concentration of each analyte using a curve fit based on the values from the reactions of the calibrator solutions. Fit each value-analyte concentration pair to a four parameter logistic binding curve to determine curve parameters. Use the values measured from each of the test samples and the parameters determined from the curve fit to calculate the concentration of each analyte from each test sample.

Referring to Fig. 1, the apparatus for practicing the method of the instant invention, generally designated by the numeral 10, comprises an inlet 12 for a test sample which is to be analyzed; an inlet 14 for a reagent mixture containing a plurality of types of sensor particles, each type of sensor particle being designed to specifically interact with an analyte of interest to vary the optical properties of an associated fluorescent indicator; an incubation chamber or vessel 16 in which the sample and reagent mixture are allowed to interact; a flow cytometer read cell 18 adapted to read coding indicia on each sensor particle passed through the cell to identify the type of sensor particle and the analyte of interest with which the particle interacts, and to read the associated fluorescent indicator to obtain a measured value indicative of whether the sensor particle has interacted with the respective analyte of interest; and a controller 20 adapted to store measured values read for each sensor particle according to the type of sensor particle and the specific analyte with which that type of sensor particle - interacts, and to process the stored measured values for each sensor particle type to obtain an assay result for the analyte associated with each type of sensor particle.

The flow cytometer read cell 18 comprises a flow cell 22 and suitable diode lasers 24 which excite respective bead labels and fluorescent indicators and photodetectors 26 which detect the resulting signals and pass them on to controller 20. The diode lasers and photodetectors preferably comprise a first diode laser which excites the bead label of each bead to emit an identifying fluorescent signal and a first photodetector which detects the identifying fluorescent signal; and a second laser diode which excites the fluorescent indicator associated with each bead to emit a measurement signal and a second photodetector which detects the measurement signal. The measurement signal is preferably in the near-infrared wavelength range.

The foregoing description and examples have been set forth merely to illustrate the invention and are not intended to be limiting.

## Claims

1. A reagent for measuring target analytes in a test sample, said reagent comprising at least one type of analyte sensor particle selected from each of the following classes (a) and (b):
(a) ion-sensors comprising a plurality of sample-insoluble particles having associated therewith a target ionophore adapted to interact with a target ion in the sample, and a first emitted fluorescent signal following interaction of the target ionophore with a target ion;
(b) metabolite-sensors comprising a plurality of sample-insoluble particles having associated therewith a ligand adapted to interact with a target metabolite in the sample to produce a second fluorescent signal following interaction of the ligand with the target metabolite;
and at least one type of analyte sensor particle selected from the following classes (c), (d) and (e):
(c) enzyme-sensors comprising a plurality of sample-insoluble particles having associated therewith a fluorogenic substrate adapted to interact with a target enzyme in the sample to produce a third fluorescent signal;
(d) antigen- or antibody-sensors comprising a plurality of sample-insoluble particles having associated therewith an immobilized pair member adapted to interact to form a complex with a complementary target antigen or a complementary antibody and to produce a fourth fluorescent signal following interaction of the pair member with the complementary target antigen or complementary antibody; and
(e) nucleotide sequence-sensors comprising a plurality of sample-insoluble particles having associated therewith a polynucleotide molecule complementary to a target nucleotide sequence and capable of hybridizing with the target nucleotide sequence under hybridizing conditions, and a fluorescent signal material that produces a fifth fluorescent signal upon hybridization between the complementary polynucleotide molecule and the target nucleotide sequence;
wherein the reagent mixture includes sensor particles which interact specifically with each of the following analytes:
(A) at least one analyte selected from the group consisting of alkali metal ions, alkaline earth metal ions, ammonium, halide ions, oxygen, pH, carbon dioxide;
(B) at least one analyte selected from the group consisting of saccharides, ammonia, urea, uric acid, cholesterol, triglycerides, ethanol, lactate, salicylate, acetaminophen, bilirubin and creatinine; enzymes, antibodies, antigens and polynucleotide sequences.

2. A reagent according to claim 1, wherein said alkali metal ions are selected from the group consisting of ions of sodium and potassium; said alkaline earth metal ions are selected from the group consisting of ions of calcium and magnesium; and said halide ions are chloride ions.

3. A reagent according to claim 1, wherein said saccharides are selected from the group consisting of glucose, fructose, lactose and galactose.

4. A reagent according to claim 1, wherein said enzymes are selected from the group consisting of alkaline phosphatase, alanine aminotransferase, aspartate aminotransferase, amylase, cholinesterase, creatine kinase, gamma-glutamyl transferase, lactate dehydrogenase and lipase.

5. A reagent according to any of claims 1 to 4, wherein said sensor particles have a size in the range from about 0.1 µm to about 50 µm.

6. A reagent according to any of claims 1 to 5, which comprises at least one type of analyte sensor particle selected from each of the classes (a), (b) and (c).

7. A reagent according to any of claims 1 to 5, which comprises at least one type of analyte sensor particle selected from each of the classes (a), (b) and (d).

8. A reagent according to any of claims 1 to 5, which comprises at least one type of analyte sensor particle selected from each of the classes (a), (b) and (e).

9. A reagent according to any of claims 1 to 5, which comprises at least one type of analyte sensor particle selected from each the classes (a), (b), (c) and (d).

10. A reagent according to any of claims 1 to 5, which comprises at least one type of analyte sensor particle selected from each of the classes (a), (b), (c) and (e).

11. A reagent according to any of claims 1 to 5, which comprises at least one type of analyte sensor particle selected from each of the classes (a), (b), (d) and (e).

12. A reagent according to any of claims 1 to 5, which comprises at least one type of analyte sensor particle selected from each of the classes (a), (b) , (c), (d) and (e).

13. A method for assaying multiple analytes in a test sample, said method comprising the steps of:
(1) admixing a test sample containing multiple analytes to be measured with a reagent mixture comprising a plurality of types of sensor particles, each type of sensor particle comprising coding indicia which confer uniquely identifying optical properties on that type of particle and a measurement substrate which specifically interacts with an analyte of interest such that measurement optical properties of said substrate are varied, said reagent mixture including particles which interact specifically with each of the following analytes:
(A) at least one analyte selected from the group consisting of alkali metal ions, alkaline earth metal ions, ammonium, halide ions, oxygen, pH, carbon dioxide;
(B) at least one analyte selected from the group consisting of saccharides, ammonia, urea, uric acid, cholesterol, triglycerides, ethanol, lactate, salicylate, acetaminophen, bilirubin and creatinine;
(C) at least one analyte selected from the group consisting of enzymes, antibodies, antigens, and polynucleotide sequences,
(2) allowing the resulting admixture to incubate for a period of time sufficient for each type of sensor particle to interact with the analyte with which it specifically interacts to vary the measurement optical properties of the sensor particle;
(3) transferring the admixture to a reading device and reading both the coding and the measurement optical properties of each sensor particle individually;
(4) storing the measured optical properties of each sensor particle type according to the coding optical properties read from the particles; and
(5) processing the stored measurements for each sensor particle type to obtain an assay result for the analyte associated with each type of sensor particle;
whereby a complete chemical analysis of the test sample is obtained.

14. A method according to claim 13, wherein said alkali metal ions are selected from the group consisting of ions of sodium and potassium; said alkaline earth metal ions are selected from the group consisting of ions of calcium and magnesium; and said halide ions are chloride ions.

15. A method according to claim 13 or 14, wherein said saccharides are selected from the group consisting of glucose, fructose, lactose and galactose.

16. A method according to any of claims 13 to 15, wherein said enzymes are selected from the group consisting of alkaline phosphatase, alanine aminotransferase, aspartate aminotransferase, amylase, cholinesterase, creatine kinase, gamma-glutamyl transferase, lactate dehydrogenase and lipase.

17. A method according to any of claims 13 to 16, wherein said reading device is a flow cytometer, said measurement optical properties of said particles are fluorescence, and said reading step is carried out by measuring the fluorescence of each type of sensor particle.

## Patentansprüche

1. Reagenz bzw. Mittel zum Messen von Ziel-Analyten in einer Testprobe, wobei das Mittel mindestens einen Analyt-Sensorteilchentyp umfaßt, ausgewählt aus jeder der folgenden Klassen (a) und (b):
(a) Ionen-Sensoren, umfassend eine Vielzahl von in der Probe unlöslichen Teilchen, welche daran assoziiert ein Ziel-lonophor aufweisen, welches so angepaßt ist, daß es mit einem Ziel-Ion in der Probe wechselwirkt und ein zuerst emittiertes Fluoreszenzsignal, welches auf die Wechselwirkung des Ziel-lonophors mit dem Ziel-Ion folgt;
(b) Metabolit-Sensoren, umfassend eine Vielzahl von in der Probe unlöslichen Teilchen, welche daran assoziiert einen Liganden aufweisen, welcher so angepaßt ist, daß er mit einem Ziel-Metabolit in der Probe wechselwirkt, um ein zweites Fluoreszenzsignal herzustellen, welches auf die Wechselwirkung des Liganden mit dem Ziel-Metabolit folgt;
und mindestens einen Analyt-Sensorteilchentyp, ausgewählt aus den folgenden Klassen (c), (d) und (e):
(c) Enzym-Sensoren, umfassend eine Vielzahl von in der Probe unlöslichen Teilchen, welche daran assoziiert ein fluorogenes Substrat aufweisen, welches so angepaßt ist, daß es mit einem Ziel-Enzym in der Probe wechselwirkt, daß es ein drittes Fluoreszenzsignal erzeugt;
(d) Antigen- oder Antikörper-Sensoren, umfassend eine Vielzahl von in der Probe unlöslichen Teilchen, welche daran assoziiert ein immobilisiertes Paarmitglied aufweisen, welches so angepaßt ist, daß es einen Komplex mit einem komplementären Ziel-Antigen oder einem komplementären Antikörper bildet, und daß es ein viertes Fluoreszenzsignal erzeugt, welches auf die Interaktion des Paarmitglieds mit dem komplementären Ziel-Antigen oder dem komplementären Antikörper folgt; und
(e) Nukleotidsequenz-Sensoren, umfassend eine Vielzahl von in der Probe unlöslichen Teilchen, welche damit assoziiert ein Polynukleotidmolekül aufweisen, welches komplementär zu einer Ziel-Nukleotidsequenz ist, und welches befähigt ist, mit der Ziel-Nukleotidsequenz unter Hybridisierungsbedingungen zu hybridisieren, und ein Fluoreszenzsignalmaterial, das ein fünftes Fluoreszenzsignal nach der Hybridisation zwischen dem komplementären Polynukleotidmolekül und der Ziel-Nukleotidsequenz erzeugt;
wobei das Gemisch der Mittel Sensorteilchen enthält, welche spezifisch mit einem der folgenden Analyte wechselwirken:
(A) mindestens ein Analyt, ausgewählt aus der Gruppe, bestehend aus Alkalimetallionen, Erdalkalimetallionen, Ammonium, Halogenididionen, Sauerstoff, pH, Kohlendioxid;
(B) mindestens ein Analyt, ausgewählt aus der Gruppe, bestehend aus Sacchariden, Ammoniak, Harnstoff, Harnsäure, Cholesterol, Triglyceriden, Ethanol, Lactat, Salicylat, Acetaminophen, Bilirubin und Kreatinin; Enzymen, Antikörpern, Antigenen und Polynukleotidsequenzen.

2. Mittel nach Anspruch 1, wobei die Alkalimetallionen ausgewählt sind aus der Gruppe, bestehend aus Ionen von Natrium und Kalium; die Erdalaklimetallionen, ausgewählt sind aus der Gruppe, bestehend aus Ionen von Calcium und Magnesium; und die Halogenidionen Chloridionen sind.

3. Mittel nach Anspruch 1, wobei die Saccharide ausgewählt sind aus der Gruppe, bestehend aus Glucose, Fructose, Lactose und Galactose.

4. Mittel nach Anspruch 1, wobei die Enzyme ausgewählt sind aus der Gruppe, bestehend aus alkalischer Phosphatase, Alaninaminotransferase, Aspartataminotransferase, Amylase, Cholinesterase, Kreatinkinase, Gamma-Glutamyl-Transferase, Lactatdehydrogenase und Lipase.

5. Mittel nach einem der Ansprüche 1 bis 4, wobei die Sensorteilchen eine Größe im Bereich von etwa 0,1 µm bis etwa 50 µm aufweisen.

6. Mittel nach einem der Ansprüche 1 bis 5, welches mindestens einen Analyt-Sensorteilchenzyp, ausgewählt aus jeder der Klassen (a), (b) und (c), umfaßt.

7. Mittel nach einem der Ansprüche 1 bis 5, welches mindestens einen Analyt-Sensorteilchentyp, ausgewählt aus jeder der Klassen (a), (b) und (d), umfaßt.

8. Mittel nach einem der Ansprüche 1 bis 5, welches mindestens einen Analyt-Sensorteilchentyp, ausgewählt aus jeder der Klassen (a), (b) und (e), umfaßt.

9. Mittel nach einem der Ansprüche 1 bis 5, welches mindestens einen Analyt-Sensorteilchentyp, ausgewählt aus jeder der Klassen (a), (b), (c) und (d), umfaßt.

10. Mittel nach einem der Ansprüche 1 bis 5, welches mindestens einen Analyt-Sensorteilchentyp, ausgewählt aus jeder der Klassen (a), (b), (c) und (e), umfaßt.

11. Mittel nach einem der Ansprüche 1 bis 5, welches mindestens einen Analyt-Sensorteilchentyp, ausgewählt aus jeder der Klassen (a), (b), (d) und (e), umfaßt.

12. Mittel nach einem der Ansprüche 1 bis 5, welches mindestens einen Analyt-Sensorteilchentyp, ausgewählt aus jeder der Klassen (a), (b), (c), (d) und (e), umfaßt.

13. Verfahren zum Testen mehrerer Analyte in einer Testprobe, wobei das Verfahren die Schritte umfaßt:
(1) Beimischen einer Testprobe, welche verschiedene, zu messende Analyte enthält, mit einem Gemisch aus Mitteln, welche eine Vielzahl von Sensorteilchentypen umfaßt, wobei jeder Sensorteilchentyp kodierende Markierungen umfaßt, welche diesem Teilchentyp einzigartige identifizierende optische Eigenschaften verleiht, und ein Meßsubstrat, welches spezifisch mit dem Analyt des Interesses wechselwirkt, so daß das Messen der optischen Eigenschaften des Substrats variiert, wobei das Gemisch an Mitteln Teilchen enthält, welche spezifisch mit jedem der folgenden Analyte wechselwirken:
(A) mindestens ein Analyt, ausgewählt aus der Gruppe, bestehend aus Alkalimetallionen, Erdalkalimetallionen, Ammonium, Halogenidionen, Sauerstoff, pH, Kohlendioxid;
(B) mindestens ein Analyt, ausgewählt aus der Gruppe, bestehend aus Sacchariden, Ammoniak, Harnstoff, Harnsäure, Cholesterol, Triglyceriden, Ethanol, Lactat, Salicylat, Acetaminophen, Bilirubin und Kreatinin;
(C) mindestens ein Analyt, ausgewählt aus der Gruppe, bestehend aus Enzymen, Antikörpern, Antigenen und Polynukleotidsequenzen,
(2) Ermöglichen, daß die resultierende Beimischung für einen Zeitraum, der ausreicht, damit jeder Sensorteilchentyp mit dem Analyt wechselwirkt, mit dem er spezifisch wechselwirkt, um die optischen Meßeigenschaften des Sensorteilchens zu variieren;
(3) Übertragen des Gemisches auf eine Lesevorrichtung und individuelles Auslesen von sowohl den kodierenden als auch den optischen Meßeigenschaften von jedem Sensorteilchen;
(4) Speichern der optischen Meßeigenschaften von jedem Sensorteilchen, gemäß den optischen kodierenden Eigenschaften, welche von den Teilchen ausgelesen werden; und
(5) Verarbeiten der gespeicherten Messungen für jeden Sensorteilchentyp, um ein Testergebnis für den mit jedem Sensorteilchen assoziierten Analyt zu erhalten;
wobei eine vollständige chemische Analyse der Testprobe erhalten wird.

14. Verfahren nach Anspruch 13, wobei die Alkalimetallionen ausgewählt sind aus der Gruppe, bestehend aus Ionen von Natrium und Kalium; die Erdalkalimetallionen ausgewählt sind aus der Gruppe, bestehend aus Ionen von Calcium und Magnesium; und die Halogenidionen Chloridionen sind.

15. Verfahren nach Anspruch 13 oder 14, wobei die Saccharide ausgewählt sind aus der Gruppe, bestehend aus Glucose, Fructose, Lactose und Galactose.

16. Verfahren nach einem der Ansprüche 13 bis 15, wobei die Enzyme ausgewählt sind aus der Gruppe, bestehend aus alkalischer Phosphatase, Alaninaminotransferase, Aspartataminotransferase, Amylase, Cholinesterase, Kreatinkinase, Gamma-Glutamyl-Transferase, Lactatdehydrogenase und Lipase.

17. Verfahren nach einem der Ansprüche 13 bis 16, wobei die Lesevorrichtung ein Durchflußcytometer ist, wobei die optischen Meßeigenschaften der Teilchen Fluoreszenz sind, und wobei der Ausleseschritt durch das Messen der Fluoreszenz von jedem Sensorteilchentyp ausgeführt wird.

## Revendications

1. Réactif pour mesurer des substances cibles à analyser dans un échantillon d'essai, ledit réactif comprenant au moins un type de particule de capteur de substance à analyser, choisi dans chacune des classes (a) et (b) suivantes :
(a) des capteurs d'ions comprenant une pluralité de particules insolubles dans un échantillon, auxquels est associé un ionophore cible adapté de façon à réagir avec un ion cible dans l'échantillon, et un premier signal fluorescent émis après réaction de l'ionophore cible avec un ion cible ;
(b) des capteurs de métabolites comprenant une pluralité de particules insolubles dans un échantillon, auxquels est associé un ligand adapté de façon à réagir avec un métabolite cible dans l'échantillon pour produire un second signal fluorescent après réaction du ligand avec le métabolite cible ;
et au moins un type de particule de capteur de substance à analyser, choisi parmi les classes (c), (d) et (e) suivantes :
(c) des capteurs d'enzymes comprenant une pluralité de particules insolubles dans un échantillon, auxquels est associé un substrat fluorogène adapté de façon à réagir avec une enzyme cible dans l'échantillon pour produire un troisième signal fluorescent ;
(d) des capteurs d'antigènes ou d'anticorps comprenant une pluralité de particules insolubles dans un échantillon, auxquels est associé un élément immobilisé d'une paire adapté de façon à réagir pour former un complexe avec un antigène cible complémentaire ou un anticorps complémentaire et pour produire un quatrième signal fluorescent après réaction de l'élément de la paire avec l'antigène cible complémentaire ou l'anticorps complémentaire ; et
(e) des capteurs de séquences nucléotidiques comprenant une pluralité de particules insolubles dans un échantillon, auxquels est associée une molécule de polynucléotide complémentaire d'une séquence nucléotidique cible et capable de s'hybrider avec la séquence nucléotidique cible dans des conditions d'hybridation, et une matière de signal fluorescent qui produit un cinquième signal fluorescent par hybridation entre la molécule de polynucléotide complémentaire et la séquence nucléotidique cible ;
dans lequel le mélange de réactif comprend des particules de capteurs qui réagissent spécifiquement avec chacune des substances suivantes à analyser :
(A) au moins une substance à analyser choisie dans l'ensemble constitué d'ions de métaux alcalins, d'ions de métaux alcalino-terreux, de l'ammonium, d'ions halogénures, de l'oxygène, du pH, du dioxyde de carbone ;
(B) au moins une substance à analyser choisie dans l'ensemble constitué de saccharides, l'ammoniac, l'urée, l'acide urique, le cholestérol, les triglycérides, l'éthanol, un lactate, un salicylate, l'acétaminophène, la bilirubine et la créatinine ; des enzymes, des anticorps, des antigènes et des séquences polynucléotidiques.

2. Réactif selon la revendication 1, dans lequel lesdits ions de métaux alcalins sont choisis dans l'ensemble constitué d'ions de sodium et de potassium ; lesdits ions de métaux alcalino-terreux sont choisis dans l'ensemble constitué d'ions de calcium et de magnésium ; et lesdits ions halogénures sont des ions chlorures.

3. Réactif selon la revendication 1, dans lequel lesdits saccharides sont choisis dans l'ensemble constitué du glucose, du fructose, du lactose et du galactose.

4. Réactif selon la revendication 1, dans lequel lesdites enzymes sont choisies dans l'ensemble constitué de la phosphatase alcaline, l'alanine-aminotransférase, l'aspartate-aminotransférase, l'amylase, la cholinestérase, la créatine-kinase, la gamma-glutamyltransférase, la lactate-déshydrogénase et la lipase.

5. Réactif selon l'une quelconque des revendications 1 à 4, dans lequel lesdites particules de capteurs ont une taille d'environ 0,1 µm à environ 50 µm.

6. Réactif selon l'une quelconque des revendications 1 à 5, comprenant au moins un type de particule de capteur de substance à analyser choisi parmi chacune des classes (a), (b) et (c).

7. Réactif selon l'une quelconque des revendications 1 à 5, comprenant au moins un type de particule de capteur de substance à analyser choisi parmi chacune des classes (a), (b) et (d).

8. Réactif selon l'une quelconque des revendications 1 à 5, comprenant au moins un type de particule de capteur de substance à analyser choisi parmi chacune des classes (a), (b) et (e).

9. Réactif selon l'une quelconque des revendications 1 à 5, comprenant au moins un type de particule de capteur de substance à analyser choisi parmi chacune des classes (a), (b), (c) et (d).

10. Réactif selon l'une quelconque des revendications 1 à 5, comprenant au moins un type de particule de capteur de substance à analyser choisi parmi chacune des classes (a), (b), (c) et (e).

11. Réactif selon l'une quelconque des revendications 1 à 5, comprenant au moins un type de particule de capteur de substance à analyser choisi parmi chacune des classes (a), (b), (d) et (e).

12. Réactif selon l'une quelconque des revendications 1 à 5, comprenant au moins un type de particule de capteur de substance à analyser choisi parmi chacune des classes (a), (b), (c), (d) et (e).

13. Procédé d'analyse de substances multiples à analyser dans un échantillon d'essai, ledit procédé comprenant les étapes consistant à :
(1) mélanger un échantillon d'essai contenant des substances multiples à analyser et à mesurer avec un mélange de réactif comprenant une pluralité de types de particules de capteurs, chaque type de particule de capteur comprenant des indices de codage qui confèrent de façon unique une identification des propriétés optiques de ce type de particule et un substrat de mesure qui réagit spécifiquement avec une substance à analyser présentant un intérêt, de manière telle que les propriétés optiques de mesure dudit substrat varient, ledit mélange de réactif comprenant des particules qui réagissent spécifiquement avec chacune des substances suivantes à analyser :
(A) au moins une substance à analyser choisie dans l'ensemble constitué d'ions de métaux alcalins, d'ions de métaux alcalino-terreux, de l'ammonium, d'ions halogénures, de l'oxygène, du pH, du dioxyde de carbone ;
(B) au moins une substance à analyser choisie dans l'ensemble constitué de saccharides, l'ammoniac, l'urée, l'acide urique, le cholestérol, les triglycérides, l'éthanol, un lactate, un salicylate, l'acétaminophène, la bilirubine et la créatinine ;
(C) au moins une substance à analyser choisie dans l'ensemble constitué d'enzymes, d'anticorps, d'antigènes et de séquences polynucléotidiques,
(2) laisser le mélange résultant incuber pendant un laps de temps suffisant pour que chaque type de particule de capteur réagisse avec la substance à analyser avec laquelle elle réagit spécifiquement de façon à modifier les propriétés optiques de mesure de la particule de capteur ;
(3) transférer le mélange dans un dispositif de lecture et lire à la fois le codage et les propriétés optiques de mesure de chaque particule de capteur individuellement ;
(4) stocker les propriétés optiques mesurées de chaque type de particule de capteur conformément aux propriétés optiques de codage lues à partir des particules ; et
(5) traiter les mesures stockées pour chaque type de particule de capteur pour obtenir un résultat d'analyse pour la substance à analyser associée à chaque type de particule de capteur ;
moyennant quoi on obtient une analyse chimique complète de l'échantillon d'essai.

14. Procédé selon la revendication 13, dans lequel lesdits ions de métaux alcalins sont choisis dans l'ensemble constitué d'ions de sodium et de potassium ; lesdits ions de métaux alcalino-terreux sont choisis dans l'ensemble constitué d'ions de calcium et de magnésium ; et lesdits ions halogénures sont des ions chlorures.

15. Procédé selon la revendication 13 ou 14, dans lequel lesdits saccharides sont choisis dans l'ensemble constitué du glucose, du fructose, du lactose et du galactose.

16. Procédé selon l'une quelconque des revendications 13 à 15, dans lequel lesdites enzymes sont choisies dans l'ensemble constitué de la phosphatase alcaline, l'alanine-aminotransférase, l'aspartate-aminotransférase, l'amylase, la cholinestérase, la créatine-kinase, la gamma-glutamyltransférase, la lactate-déshydrogénase et la lipase.

17. Dispositif selon l'une quelconque des revendications 13 à 16, dans lequel ledit dispositif de lecture est un cytomètre de flux, lesdites propriétés optiques de mesure desdites particules sont la fluorescence, et ladite étape de lecture est effectuée en mesurant la fluorescence de chaque type de particule de capteur.
